# DEMANDE DE BREVET EUROPEEN

(11) **EP 0 601 908 A1**
(43) Date de publication de la demande: **15.06.1994**
(21) Numéro de dépôt: 93402868.9
(22) Date de dépôt: 26.11.1993
(51) Int. Cl.: A61K 9/20

(54) **Procédé d'obtention d'un médicament se présentant sous la forme d'un produit comprimé pouvant être avalé, sucé, croqué, délité**

(30) Priorité: 11.12.1992 FR 9214928
(71) Demandeur: Grimberg, Georges Serge, F-75007 Paris (FR)
(72) Inventeur: Grimberg, Georges Serge, F-75007 Paris (FR)
(74) Mandataire: Madeuf, Claude Alexandre Jean

(57) **Abrégé**

Procédé d'obtention d'un médicament se présentant sous la forme d'un produit comprimé pouvant être avalé, sucé, croqué, délité, caractérisé en ce qu'on mélange en proportions convenables, une gomme et un produit insoluble, le mélange compacté étant ensuite broyé et passé à travers une grille d'un maillage approprié.

## Description

La présente invention a pour objet un procédé d'obtention d'un médicament se présentant sous la forme d'un produit comprimé pouvant être avalé, sucé, croqué, délité par exemple dans de l'eau.

Cette présentation, sous une forme "per os" universelle, permet au médecin de prescrire un médicament acceptable à son patient, qu'il soit enfant, adulte ou personne âgée.

Conformément à l'invention, le procédé se caractérise par le fait qu'on mélange, en proportions convenables, une gomme et un produit insoluble, le mélange compacté étant ensuite broyé et passé à travers une grille d'un maillage approprié.

Suivant une autre caractéristique de l'invention, le procédé s'étend également à un mélange de produits pharmaceutiques présenté sous la forme d'un produit compacté, broyable et passé à travers une grille à mailles, ce mélange ayant des activités thérapeutiques complémentaires permettant d'obtenir finalement un comprimé présentant l'avantage de pouvoir être avalé, sucé, croqué, délité par exemple dans de l'eau.

L'invention s'étend également au produit obtenu par le procédé de l'invention.

On donne ci-après, à titre d'exemples non limitatifs, des formes de réalisation de produits qui seront vendus dans le commerce sous la marque "Gomacté".

### Exemple n° 1 :

Le calcium est une thérapeutique très importante à tous les âges et notamment pour la femme à partir d'un certain âge.

La gomme choisie est de la gomme de guar enrobée par du siméthicone (voir EP-A-0 194 202) ; l'insoluble est du carbonate de calcium.

| Partie A | Par comprimé |
|---|---|
| - Carbonate de calcium | 600 mg |
| - Gomme de guar enrobée | 100 mg |
| - Stéarate de magnésium | 0.3 mg |

La partie A est mélangée, compactée, puis broyée par passage à travers une grille de 2 mm de maille.

| Partie B | Par comprimé |
|---|---|
| - Gluconate de calcium 1H₂O | 6,3 mg |
| - Glucoheptonate de calcium 2H₂O | 6,3 mg |
| - Lactate de calcium 5H₂O | 6,3 mg |
| - Chlorure de calcium 2H₂O | 6,3 mg |
| - Stéarate de magnésium | 0,3 mg |

La partie B est mélangée, compactée, puis broyée par passage à travers une grille de 2 mm de maille.

| Partie C | Par comprimé |
|---|---|
| - Levure sèche | 15 mg |
| - Cyclamate de calcium | 9 mg |
| - Saccharinate de sodium | 2,5 mg |
| - Arôme citron - mirabelle | 15 mg |
| - Stéarate de magnésium | 9 mg |

Les parties A, B et C sont mélangées. Le mélange obtenu permet de fabriquer un comprimé contenant 250 mg de calcium-élément.

Le produit ainsi fabriqué peut être avalé, sucé, croqué ou délité dans un verre d'eau.

L'exemple n° 1 démontre que le produit calcium est fabriqué avec de la gomme de guar enrobée, associée à de l'insoluble : le carbonate de calcium, le tout étant compacté puis passé sur un granulateur oscillant.

Pour compléter le dosage de calcium, on ajoute du calcium soluble et ensuite on aromatise et édulcore. La levure permet une meilleure absorption du calcium, voir EP-A-0 113 608.

Il doit être noté que le stéarate de magnésium facilite l'écoulement des poudres.

### Exemple n° 2 :

Un autre exemple : le produit aspirine.

| Partie A | Par comprimé |
|---|---|
| - Aspirine | 500 mg |
| - Gomme de guar enrobée | 85 mg |

La partie A est mélangée, compactée, puis broyée par passage à travers une grille de 2 mm de maille.

| Partie B | Par comprimé |
|---|---|
| - Saccharinate de sodium | 15 mg |
| - Arôme de menthe | 15 mg |
| - Talc | 5 mg |

La partie B est mélangée, compactée, puis broyée par passage à travers une grille de 2 mm de maille.

Les parties A et B sont mélangées et servent à fabriquer un comprimé.

Le produit ainsi fabriqué peut être avalé, sucé, croqué ou délité.

### Exemple n° 3 :

Un autre exemple, il s'agit ici d'un produit anti-acide.

La formule du produit anti-acide est donnée dans le EP-A-0 194 202, et grâce à la présente invention du produit final, cet anti-acide peut être avalé, sucé, croqué ou délité parfaitement.

La gomme choisie est la gomme de guar enrobée par du siméthicone (EP-A-0 194 202), l'insoluble est de l'hydroxyde de magnésium, de l'hydroxyde d'aluminium, du phosphate d'aluminium.

| Partie A | Par comprimé |
|---|---|
| - Hydroxyde de magnésium | 250 mg |
| - Hydroxyde d'aluminium | 250 mg |
| - Phosphate d'aluminium | 150 mg |
| - Gomme de guar enrobée | 100 mg |
| - Stéarate de magnésium | 8 mg |

La partie A est mélangée, compactée, puis broyée par passage à travers une grille de 2 mm de maille.

| Partie B | Par comprimé |
|---|---|
| - Cyclamate de sodium | 15 mg |
| - Saccharinate de sodium | 7,5 mg |
| - Arôme de menthe | 10 mg |
| - Stéarate de magnésium | 9 mg |

La partie B est mélangée, puis on ajoute la partie A et l'on fabrique un comprimé.

Le "Gomacté" anti-acide ainsi fabriqué peut être avalé, sucé, croqué ou délité.

Si le médicament a pour principe actif une substance soluble, il suffit de la mélanger à un produit obtenu suivant le procédé de préparation de l'invention dit "Gomacté", c'est-à-dire à une gomme associée à de l'insoluble, compacté et broyé.

Il est à noter que le compacté doit être broyé par passage à travers une grille d'au moins 2 mm de maille.

## Revendications

**1 -** Procédé d'obtention d'un médicament se présentant sous la forme d'un produit comprimé pouvant être avalé, sucé, croqué, délité, caractérisé en ce qu'on mélange, en proportions convenables, une gomme et un produit insoluble, le mélange compacté étant ensuite broyé et passé à travers une grille d'un maillage approprié.

**2 -** Procédé suivant la revendication 1, caractérisé en ce qu'il s'étend également à un mélange de produits pharmaceutiques présenté sous la forme d'un produit compacté, broyable et passé à travers une grille à mailles, ce mélange ayant des activités thérapeutiques complémentaires permettant d'obtenir finalement un comprimé présentant l'avantage de pouvoir être avalé, sucé, croqué, délité, par exemple dans de l'eau.

**3 -** Procédé suivant la revendication 1, caractérisé en ce que la gomme est de la gomme de guar enrobée de siméthicone.

**4 -** Procédé suivant les revendications 1 et 3, caractérisé en ce que l'insoluble est n'importe quel principe actif ou excipient insoluble.

**5 -** Procédé suivant les revendications 1 à 4, caractérisé en ce qu'on utilise un mélange de gomme, d'insoluble, compacté puis passé au granulateur, grille à mailles de dimensions variables et mélangé à un ou plusieurs principes actifs solubles ou insolubles, de l'arôme et de l'édulcorant.

**6 -** Procédé d'obtention d'un médicament se présentant sous la forme d'un produit comprimé pouvant être avalé, sucé, croqué, délité sensiblement tel que décrit.
